# EUROPEAN PATENT SPECIFICATION

(11) **EP 3 915 463 B1**
(45) Date of publication and mention of the grant of the patent: **30.11.2022**
(21) Application number: 20177487.4
(22) Date of filing: 29.05.2020
(51) Int. Cl.: A61B 5/00, A61N 5/06

(54) **SYSTEM FOR DISTRIBUTING RADIATION FOR DIAGNOSTICS**
SYSTEM ZUR VERTEILUNG VON STRAHLUNG FÜR DIE DIAGNOSTIK
SYSTÈME DE DISTRIBUTION DE RAYONNEMENT POUR DES DIAGNOSTICS

(43) Date of publication of application: 01.12.2021
(73) Proprietor: SpectraCure AB, 222 29 Lund (SE)
(72) Inventor: Swartling, Johannes, 226 51 Lund (SE); Soto Thompson, Marcelo, 24798 Genarp (SE); Grönlund, Rasmus, 247 56 Dalby (SE)
(74) Representative: KIPA AB

(56) References cited:
- EP-A1- 1 624 803
- EP-B1- 1 624 803
- WO-A2-2004/012589
- US-A- 5 304 173

## Description

### BACKGROUND OF THE INVENTION

### Field of the Invention

This disclosure pertains in general to a system and a method for photodynamic therapy and/or photothermal therapy and/or diagnosis of a site on and/or in a 'body' of a subject, wherein radiation is conducted to the site for reaction with the radiation, wherein the system comprises a distributor of radiation from at least one source of radiation to a reaction site, and from the reaction site to at least one radiation sensor, respectively, and wherein the reaction site preferably is a tissue site, such as a tumour site.

### Background of the Disclosure

Within the field of medical therapy of tumour diseases, several treatment modalities have been developed for treatment of malignant tumour diseases. Surgery, cytostatic treatment, treatment with ionising radiation (gamma or particle radiation), isotope therapy, and brachytherapy employing radioactive needles are examples of common treatment modalities. In spite of great progress within therapy, tumour diseases continue to account for much human suffering, and are responsible for a high percentage of deaths in Western countries. A treatment modality, photodynamic therapy (PDT), provides a complement or alternative in the treatment field. A light-activated agent, normally referred to as a sensitizer, is administered to the body percutaneously, orally or topically. It may accumulate in malignant tumours to a higher extent than in the surrounding healthy tissue. The tumour area is then irradiated with non-thermal red or infrared light, normally from a laser, leading to excitation of the sensitizer to a more energetic state. Through energy transfer from the activated sensitizer to the oxygen molecules of the tissue, singlet state oxygen and other oxidative species are formed. Singlet oxygen is known to be particularly toxic to tissue; cells are eradicated and the tissue goes in necrosis. Because of the localization of the sensitizer to tumour cells a unique selectivity is obtained, where surrounding healthy tissue is spared.

Laser hyperthermia is a related treatment modality that, instead of utilising a photosensitizer, heats the target tissue by higher laser power and causes tissue death by thermal effect.

The limited penetration in tissue of the activating light is a drawback of PDT, and similar limitations are present for laser hyperthermia. The result is that only surface tumours can be treated by surface irradiation. In order to treat thicker and deep-lying tumours, interstitial light delivery can be utilized. Here, light-conducting optical fibres are brought into the tumour using, e.g. a syringe needle, in the lumen of which a fibre has been placed.

In order to achieve an efficient treatment, several fibres may be used to ascertain that all tumour cells are subjected to a sufficient dose of light. It has been shown to be achievable to perform dose calculations of the absorptive and scattering properties of the tissue. To perform said dose calculations, it advantageous to first perform measurements of the light flux through the tissue in the region where the treatment will take place. The measurements can then be used to determine the absorptive and scattering properties of the tissue. Said measurements can be performed with a separate set of optical fibres, but it is in general preferable to use the same set of optical fibres as is used for the treatment, since fewer optical fibres then have to be inserted into the tissue in total, and the inserted fibres will cover exactly the same volume when used for measurements and for treatment. When using the same set of optical fibres for measurement as for treatment, there is a need to have some means to switch between measurement and treatment. E.g., in the patent EP 1 443 855 A1 a system is described, where multiple fibres are used for treatment as well as for measurement of the light flux which reaches a given fibre in the penetration through the tissue from the other fibres.

The patent EP 1 443 855 A1 describes a means to switch between treatment and measurement by using optical fibres and a rotating disc arrangement. In this way the correct light dose can be achieved for all parts of the tumour.

A limitation when performing interstitial illumination and measurement as described in the previous is that the rotating disc arrangement may be slow, costly to manufacture, and require extensive alignment and service. Accordingly, in patent EP 1 624 803 A1, a system and method are described, wherein a non-mechanical operation mode selector is used to direct the therapeutic radiation and/or the diagnostic radiation to the reaction site through the radiation conductors. Several non-mechanical operation mode selectors are described, such as for example an electro-optical switch based on electrically controlled refractive index variations, or an acousto-optical switch based on sound generated Bragg deflection. However, although these non-mechanical operation mode selectors are an improvement over mechanical switches, they are active components that add cost and complexity.

Mode selection by means of passive components can be achieved by using a beam splitter to align the light paths of the therapeutic radiation and the diagnostic radiation, however, when the therapeutic radiation and the diagnostic radiation are of the same wavelength, the beam splitter will lead to loss of radiation in both the therapeutic radiation and the diagnostic radiation. For example, if the beam splitter has 50/50 transmission/reflection, half of the therapeutic radiation and half of the diagnostic radiation will be lost.

Hence, new improved apparatus and methods for combining distribution of radiation and measurement in the same members would be advantageous.

As further relevant prior art, US5304173 discloses a catheter wherein optical fibers carrying laser light are mounted in a catheter for insertion into an artery.

### SUMMARY OF THE INVENTION

Accordingly, examples of the present disclosure preferably seek to mitigate, alleviate or eliminate one or more deficiencies, disadvantages or issues in the art, such as the above-identified, singly or in any combination by providing a device, system or method according to the appended patent claims for combining distribution of radiation and measurement in the same members.

Lasers, and some light emitting diodes, are known to have high radiance, i.e., high power per emitting surface area and unit solid angle. A consequence of this property is that light emitted from a laser source can be focussed at a small area even with a small numerical aperture, NA. On the other hand, light that has traversed biological tissue has typically scattered, resulting in relatively low radiance once it is emitted from said biological tissue. In order to efficiently capture the light emitted from biological tissue, it is preferable to use a light guide with relatively high cross-sectional area and that has a high NA.

The disclosure makes use of these properties of the light source and the light guide to provide a means to passively align the operation modes of distributing light to the tissue, and measurements of said tissue, without the need for a mechanical or active switching element.

According to the invention, a system for diagnosis of a subject is described. The system includes at least one diagnostic light source for emission of diagnostic light within a wavelength range of infrared, visible or ultraviolet light, the diagnostic light source emits at least one light beam. The system also includes at least one light detector, for detection of light and a plurality of optical members adapted to conduct light to and/or from a tissue site of the subject. The distal ends of the plurality of optical members are configured to be positionable at different locations at the tissue site in order to enable an effective diagnosis and/or treatment.

The system further includes that each of the optical members is configured so that at least one of the light beams from the at least one diagnostic light source is coupled into the proximal end of the plurality of optical members by means of at least one focussing optical component. The diagnostic light transmitted back from the tissue may be emitted from the proximal end of the plurality of optical members having a higher numerical aperture (NA) than the focussed beam coupled into the optical members providing an emission at least partially in a different angular sector than an angular sector of the focussed light beam, so that the diagnostic light scattered back from the tissue is detected by the at least one light detector.

According to the invention, the plurality of optical members are configured to be interstitially arranged in tissue.

In some examples of the disclosure, the system may be further configured for interactive photodynamic or photothermal therapy, and comprising at least one therapeutic light source for emission of therapeutic light within a wavelength range of infrared, visible or ultraviolet light, said therapeutic light source emitting at least one light beam which is couples into the proximal end of the optical member by means of the focussing optical component.

In some examples of the disclosure, the diagnostic light source may be the same as the therapeutic light source.

In some examples of the disclosure, the wavelength range of the diagnostic light source may be the same as the wavelength range of the therapeutic light source.

In some examples of the disclosure, the system may further include a reflective member used for coupling the light from the proximal end of the optical member to the at least one detector.

In some examples of the disclosure, the reflective member has at least one aperture for the diagnostic light to be transmitted through when travelling between the at least one diagnostic light source and the proximal end of the plurality of optical members.

In some examples of the disclosure, the aperture is at least one hole or a slit.

In some examples of the disclosure, one of the plurality of optical members may be a transmission member used for transmitting the diagnostic light to the tissue site and at least two other optical members of the plurality of optical members are receiving members for receiving backscattered light from the tissue site for detection.

In some examples of the disclosure, the transmission member may be sequentially selected between the plurality of optical members.

In some examples of the disclosure, the system may also include a plurality of modules, wherein each module comprises a light emitting part comprising at least one of the at least one diagnostic light source, a light detecting part comprising at least one of the at least one detector, one of the at least one focussing optical component, and one of the plurality of optical members.

In some examples of the disclosure, the transmission member may be sequentially selected between the plurality of optical members by sequentially switching on and off the light emitting part of the plurality of modules.

In some examples of the disclosure, the system is configured to have an open beam path between the at least one light source and the proximal end of the plurality of optical members, and between the proximal end of the plurality of optical members and the at least one light detector.

In some examples of the disclosure, the plurality of optical members may be configured to be arranged in the tissue site to perform spatially resolved measurements.

In some examples of the disclosure, at least one second focusing element may be arranged in front of the at least one detector.

In another aspect of the disclosure, a method of coupling light in and out of an optical member is described. The method may include transmitting at least one light beam within a wavelength range of infrared, visible or ultraviolet light using a light source.

The method may also include coupling the light beam into a proximal end of the optical member by means of at least one focussing optical component.

The method may further include collecting backscattered light using a distal end of the optical member and emitting the collected light at the proximal end of the optical member and wherein the light emitted by the optical member has at least partially a different angular sector than an angular sector of the light beam being coupled into the proximal end of the optical member, and detecting the collected light emitted from the proximal end of the optical member using at least one light detector.

It should be emphasized that the term "light" when used in this specification is taken to specify electromagnetic radiation of any wavelength in the electromagnetic spectrum including ultraviolet radiation, visible light, and infrared radiation.

It should also be emphasized that the disclosure is not limited to use when performing treatment of malignant tumours, but may be used in any situation where a treatment of tissue using optical members, such as optical fibres, is performed, or any situation where measurements in tissue using optical members is performed.

It should also be emphasized that the disclosure is not limited to use with lasers as light sources, but that any type of light source with sufficiently high radiance may be used.

It should also be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which examples of the disclosure are capable of will be apparent and elucidated from the following description of examples of the present disclosure, reference being made to the accompanying drawings, in which
Fig. 1 is illustrating an schematic example of a configuration in accordance with the present disclosure;
Fig. 2 is illustrating a schematic example of an arrangement for coupling light in and out of an optical member;
Fig. 3 is illustrating a schematic example of an arrangement for coupling light in and out of an optical member;
Fig. 4 is illustrating a schematic example of an arrangement for coupling light in and out of an optical member;
Fig. 5 is illustrating an example of coupling at least two light sources at different wavelengths in and out of an optical member;
Fig. 6 is illustrating an example of coupling at least two light sources at different wavelengths in and out of an optical member;
Fig. 7 is illustrating an example of coupling at least two light sources at different wavelengths in and out of an optical member; and
Fig. 8 is an example of how the previously described configurations may be arranged as modules for coupling light in and out at multiple locations;
Fig. 9 is illustrating a schematic example of an aperture in a mirror being a hole; and
Fig. 10 is illustrating a schematic example of an aperture in a mirror being a slit or two or more separate mirrors that form one or multiple slits.

### DESCRIPTION OF EXAMPLES

The following disclosure focuses on examples of the present disclosure applicable to combining distribution of radiation and measurement in the same members by using passive components. The disclosure may be applicable for photodynamic or photothermal therapy of tissues. For example, this is advantageous for accurate dosimetry of light dose, so that the intended tissue is treated but surrounding tissues are spared. However, it will be appreciated that the description is not limited to this application but may be applied to many other systems where combining distribution of radiation and measurement in the same members by using passive components is useful.

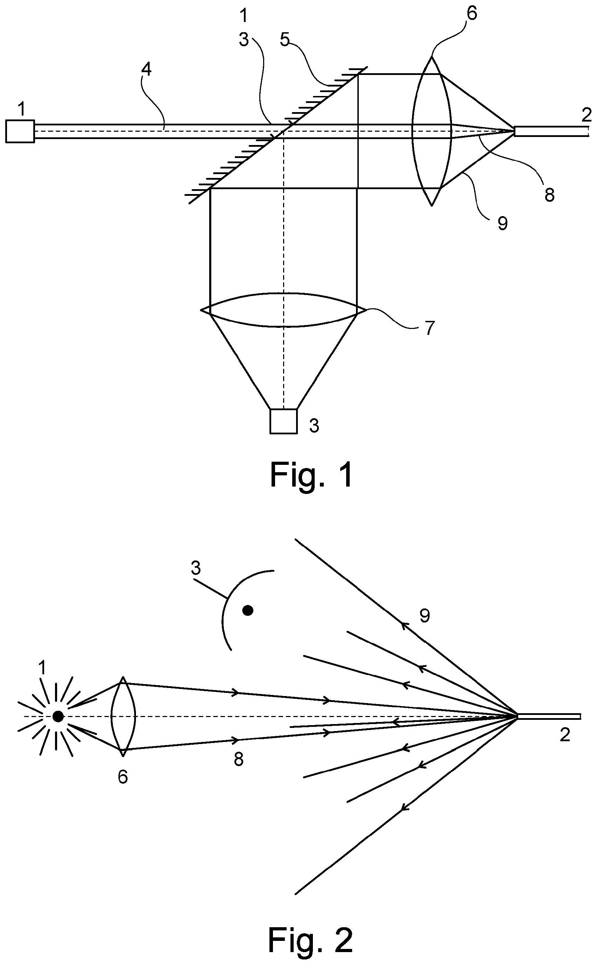

Consider a laser that emits light that is intended to be coupled into an optical member, such as an optical fibre, as illustrated in Fig. 1.

Figure 1 illustrates an example of the disclosure. A light source 1, such as a laser, may emit light 4, such as a light beam, that may be focussed by a focussing component 6, such as a lens or a lens assembly, into the optical member 2, such as an optical waveguide or an optical fibre. The light may be focused into a proximal end of the optical member 2.

Due to the properties of the light source 1, the numerical aperture NA of the focussed beam 8 may be lower than the acceptance angle of the optical member 2.

A distal end (not shown) of the optical member 2 may be used to collect back scattered light from an area to be measure or diagnosed, such as a tissue site, or a tumour. The collected back scattered light may be emitted by the proximal end of the optical member 2. The optical member 2 may have a higher NA than the focussed beam 8, and because the light collected by the optical member 2 from an area back scattering the light, such as a tissue site, is diffuse, the light 9 that is emitted from the optical member 2 fills the NA of the optical member 2. Hence, the light 9 emitted from the optical member 2 may result in a beam wider than the focused light beam 8.

The emitted light may be detected using at least one detector.

Additionally, and/or alternatively, the emitted light 4 from the at least one light source 1 may pass through a reflective member 5, such as a mirror, that allows light through an aperture 13 at a point where the light passes but would have been reflecting otherwise. Due to the light 9 emitted back from the proximal end of the optical member 2 fills the NA of the optical member 2, a major part of the light 9 emitted from the optical member 2 may reflect off the reflective member 5, and may be detected using at least one detector 3. The at least one detector may have a second focusing component 7, such as a lens or a lens assembly, arranged in front to focus the light 9 reflected by the reflective member 5 onto the detector.

The light transmitted from the at least one light source 1 to the optical member 2, and the collected light emitted from the optical member 2 to the at least one detector 3 may be transmitted in an open beam path without any use of waveguides or fibres.

With the disclosed system and method, it is possible to distribute light via the fibre and measure light, at the same wavelength, via the same fibre, without the need for a mechanical or active switch.

An advantage of using the technology in the disclosure may be that changing from light distribution to performing a measurement may be done instantaneously, which may lead to shorter time for the procedure and shorter total time in the clinic.

An additional advantage of the disclosure may be that it allows simultaneous light distribution and measurement in the same optical member, which is not possible with a switching module.

An additional advantage of the disclosure is that it has no moving parts, which may reduce the probability of component failure.

In some examples of the disclosure herein, the light 4 from the at least one light source 1 may be focussed into the proximal optical member 2 by an optical focussing component 6 by obtaining a focused light beam 8. In some examples of the disclosure herein the light emitted from the optical member may be detected directly by at least one light detector 3. The detector 3 may be, e.g., photodiodes, photomultiplier tubes, avalanche photodiodes, charge-coupled devices (CCD), or CMOS light sensitive devices.

In an example, light sources may be a lamp, photodiodes, e.g., light emitting diodes (LED) or laser diodes. The light source may have one or more filters for filtering the wavelength of the light emitted.

The light sources may be diagnostic light sources having a wavelength corresponding to the absorption of one or more chromophores in tissue, such as deoxy-hemoglobin and/or oxy-hemoglobin. The light sources may also be the same as the light sources used for treatment of the tissue site, such as treatment of a tumour.

When performing both diagnostic and treatment, they may be carried our sequentially. For example, a period of treatment may be followed by a period of diagnostic. In some example, the treatment may be carried out simultaneously as the diagnostic.

In some examples, any type of focussing component 6 is used to focus the light from the light source 1 into the optical member 2 and/or to collimate the light emitted from the proximal end of the optical member 2, such as including but not limited to lenses, curved mirrors, diffractive components, holographic components, Fresnel lenses, Fresnel mirrors, microelectromechanical (MEMS) mirrors. It should be noted that in the illustrations of the disclosure in the figures, the lens may be exchanged for any of these components.

In some example, the focussing component 7, which may be used to focus the emitted light from the optical member 2 onto the detector 3, may be but not limited to lenses, curved mirrors, diffractive components, holographic components, Fresnel lenses, Fresnel mirrors, microelectromechanical (MEMS) mirrors

In some examples, any type of optical members 2, such as light-guiding conductor may be used to conduct light to the reaction site, such as the tissue site, may include but not limited to optical fibres, liquid light guides, hollow light guides, or plastic light guides.

In some examples, the light from the light source is directed to the optical fibre by means a mirror, while the light emitted from the optical fibre is detected on the same optical axis as the optical fibre.

Consider Fig. 2, where light from a light source 1 is focussed by a an optical focussing component 6, such as a lens, to obtain a focused light beam 8 into an optical member 2, such as an optical waveguide or an optical fibre, and a portion of the light emitted by the optical member 2 may be detected by the light detector 3.

The light can be detected because the light 9 that is emitted from the optical member 2 fills the NA of the optical member 2 which may result in a beam wider than the focused light beam 8. The detector 3 may the be arranged in an angle in relation to the beam path of focused light beam 8, thereby collecting at least a portion of the light 9 that is emitted from the optical member 2.

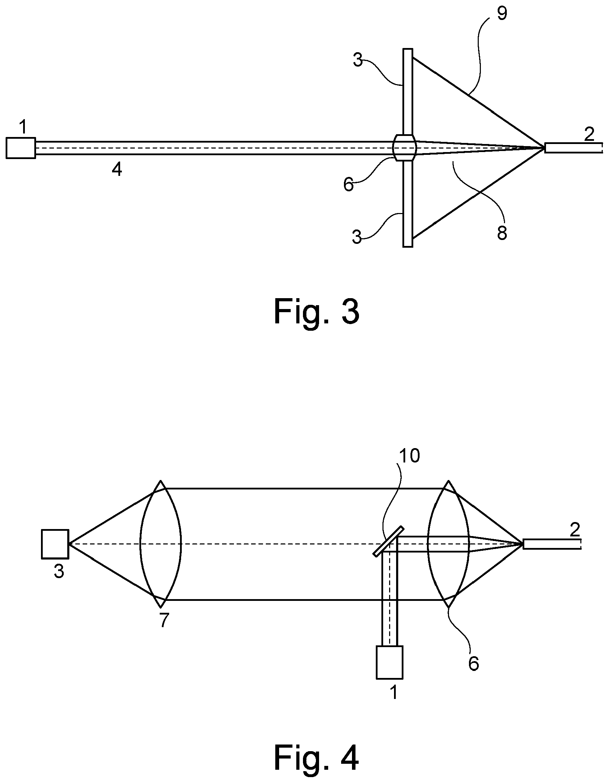

Another example is illustrated in Fig. 3, where the light beam from the light source 1 may be focussed by an optical focussing component 6, to obtain a focused light beam 8 directed into a proximal end of an optical member 2, and the light 9 emitted from the optical member 2 may be measured by a disc-shaped light detector 3 which has a hole in the centre, where the optical focussing component 6 may be positioned.

Consider Fig. 4, where the light beam from the light source 1 is reflected off a reflective member 10, such as a mirror, and focussed by a focusing component 6 into a proximal end of an optical member 2, while the light emitted from the optical member 2 is detected by a focusing component 76 and a light detector 3.

In some examples of the arrangements described herein, a plurality of light sources are coupled into the same optical member. For example, in order to measure the oxygen saturation of tissue, at least two light sources at different wavelengths are needed to distinguish between oxygenated and non-oxygenated haemoglobin. Alternatively, and/or additionally, in some examples a plurality of light sources are coupled into the same optical member, wherein at least one light source is used for measuring chromophores, such as for detection of oxygen saturation of tissue, wile at least a second light source is used for treatment.

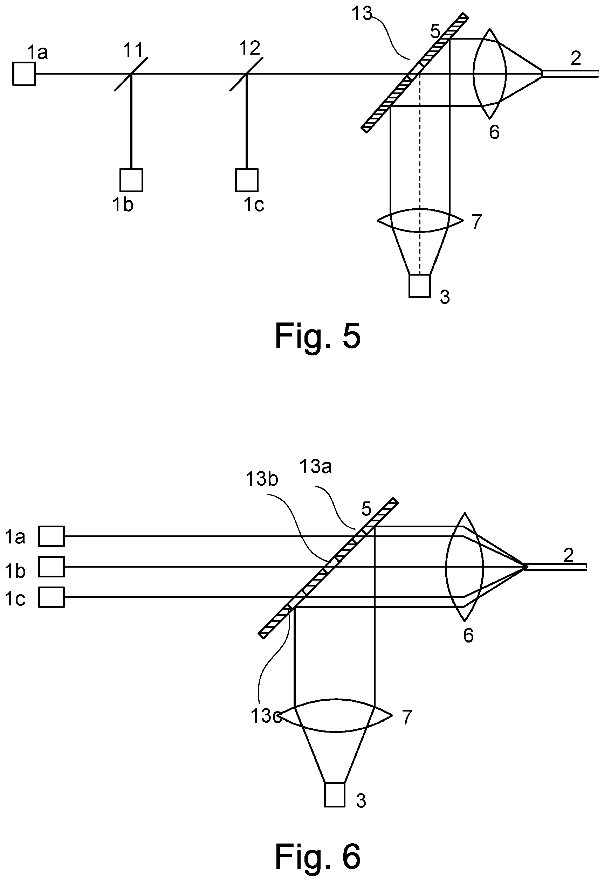

Consider Fig. 5, where at least two light sources, here illustrated as being three light sources, 1a, 1b, 1c, with different wavelengths, are coupled into the optical member 2. A beam splitter 11 may be selected so that it transmits the wavelength of light source 1a, but reflects the wavelength of light source 1b. A beam splitter 12 may be selected so that it transmits the wavelengths of light sources 1a and 1b, but reflects the wavelength of light source 1c. This may be achieved by arranging the light sources 1a, 1b, 1c, 2, and 3 with increasing wavelength, and selecting a beam splitter 11 as a short-pass filter, and beam splitter 12 as a short-pass filter with a higher cut-off wavelength.

Alternatively, it may be achieved by arranging the light sources 1a, 1b, and 1c with decreasing wavelength, and selecting a beam splitter 11 as a long-pass filter, and beam splitter 12 as a long-pass filter with a lower cut-off wavelength. It should be noted that the number of light sources is not limited to three, and that any number of light sources can be used with the same principle. It should also be noted that the beam splitters 11, 12 are not limited to high-pass or low-pass beam splitters, and that any wavelength-selective beam splitter with appropriate properties may be used, such as band-pass beam splitters or notch beam splitters.

The arrangement for coupling the light in and out of the optical member 2 is illustrated as in Fig. 1, this is only an example and other ways may be possible, such as described in relation to Figs. 2 to 4.

In some examples, a plurality of light sources 1a, 1b, 1c may be coupled into the optical member 2 without the need for beam splitters. Consider the illustration in Fig. 6, where the light, such as light beams, from each light source 1a, 1b, 1c are separated spatially and each of them passes through an aperture 13a, 13b, 13c in a reflective member 5, such as a mirror, and then focussed into the optical member 2 by a focusing component 6.

The collected light may then be emitted by the proximal end of the optical member 2, collimated by the focusing component 6, and reflected by the reflective member 5 to be directed towards at least one detector 3. The at least one detector may have a second collective component 7 arranged in front to focus the light onto the at least one detector 3.

In some examples, instead of using a single aperture 13 for each light path and/or beam, a slit may be used in the reflective member 5.

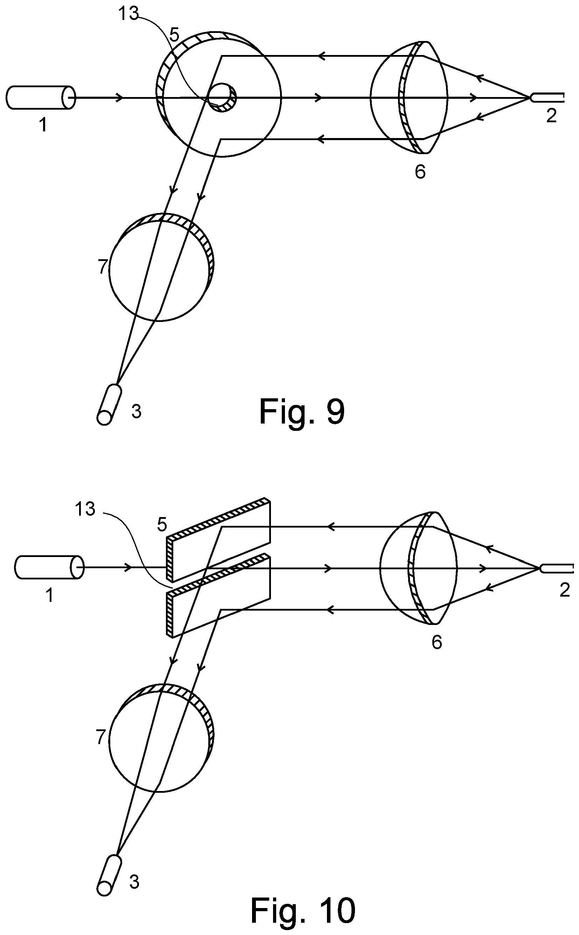

How an arrangement with a hole as an aperture 13 may look is further illustrated in Fig. 9 while the alternative of using a slit as an aperture 13 is further illustrated in Fig. 10. In these examples, the reflective member 5 is used with a single light source 1 but the principle is the same for multiple light sources. For Fig. 9, when using a plurality of light sources 1, the reflective member 5 may have additional apertures 13, such as one aperture 13 for each light source and/or light path and/or light beam. In Fig. 10 a single slit may be used with a plurality of light sources, as long as the slit is long enough to accommodate the light paths from the light sources 1. Alternatively, the reflective member 5 may have a slit 13 for each light source and/or light path and/or light beam. The slit may be obtained by either making the slit into a reflective member, such as a mirror, or by positioning two mirror next to each other with a gap in between, wherein the gab will be a slit.

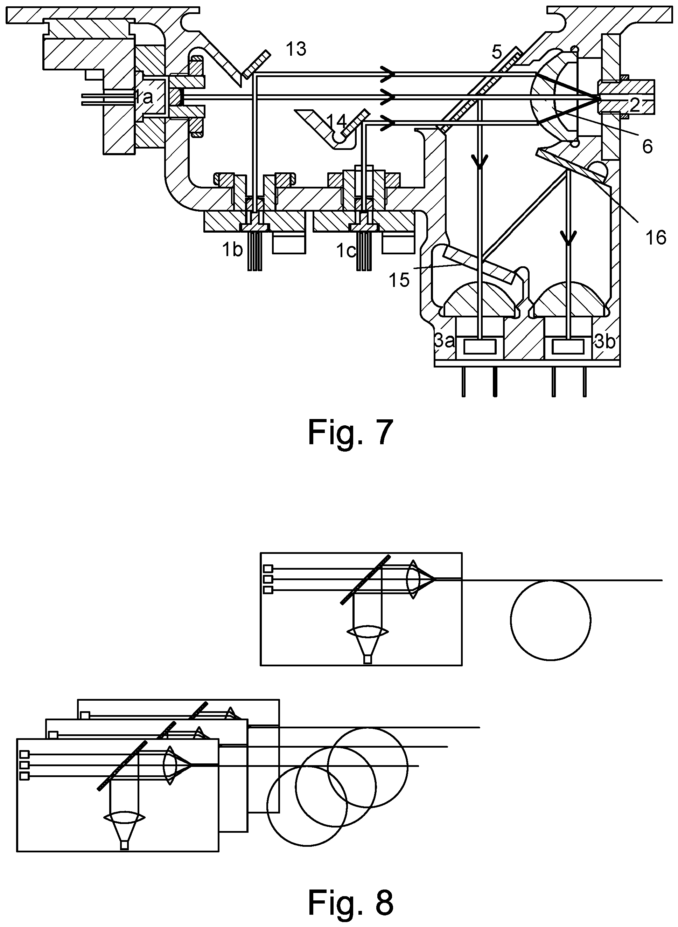

Consider the illustration in Fig. 7, where the light beams from each light source 1a, 1b, 1c may be separated spatially using reflective components 13, 14. In this example, one of the light sources 1a is arranged along an optical axis which the other two are arranged at an angle, such as perpendicular, to the optical axis. By arranging the reflective members 13, 14 at different distances from the optical axis, the light may be reflected from the other light sources 1b, 1c to obtain three parallel light paths and/or beam paths.

Each of the parallel light paths and/or beam paths may pass through an aperture, for example as illustrated in Figs. 9 or 10, in a reflective member 5. The light may then be focussed into the optical member 2 by a focusing component 6. Most of the returning light from the optical member 2 may be reflected by the reflective member 5, to the beam splitter 15, which reflects light with the wavelength of light source 1a (the shortest wavelength), while light with longer wavelength than that of light source 1a may be transmitted and may be detected by the detector 3a. The light that is reflected by the beam splitter 15 may be detected by detector 3b or may be reflected by a further reflective element 16, such as a mirror, which will direct the light towards the detector 3b. With this arrangement, light at the wavelength of light source 1a may be detected simultaneously with light from either light source 1b or 1c. Alternatively, the light from light source 1a may induce fluorescence in the tissue at wavelengths longer than the wavelength of light source 1a, for example fluorescence by a photosensitizer. In such a case, the fluorescence light may be detected by detector 3a, separated from the light from light source 1a which will be detected by detector 3b. The light sources 1b and 1c may be switched on sequentially after light source 1a and the detected light may be detected with detector 3a.

In some examples, a plurality of modules such as described in the disclosure are combined into a single complete system, so that the modules may interact in the manner described in the patent EP 1 443 855 A1. Fig. 8 illustrates a system based on a plurality of modules such as illustrated in any of Figs. 1 to 7, where a plurality of optical fibres may be connected to said system. The system may include at least two optical members configured to be inserted into tissue, such as interstitially inserted. The at least two optical members may be configured for emitting and/or collecting light. The system may further include a control unit configured for controlling the system so that light is transmitted to the tissue from at least one optical member and light is detected from the tissue by at least one optical member collecting light. This may yield a data set of measured values for pairs of emitting and collecting optical members.

For example, a transmission member of the optical members is sequentially selected between the plurality of optical members. This may be done by sequentially switching on and off the light emitting part of the plurality of modules.

In some example may the system include a plurality of optical members. In some examples one single fibre at a time may emit light, and all other fibres collecting. Alternatively, in some example other measurement regimens may be possible, such as using a sub-set of all fibres that emit, or a sub-set of all fibres that collect, or combinations of these.

In some example of the system, may the measured and/or determined optical properties of the tissue be used for calculating a light dose for photodynamic therapy or laser hyperthermia.

In some examples are the the plurality of optical members configured to be arranged on the tissue site so that a spatially resolved measurement may be performed. The optical properties may then be obtained from the measurements by solving the transport equation of radiative transfer.

In some example of the system, may the optical members be optical fibres, or optical fibres with diffusers. The optical member may be configured to be interstitially arranged in tissue to allow treatment and/or diagnosis of a deep laying tissue site. In one example, this may be done using needles, syringes and/or a catheter.

Alternatively, in some examples, the optical members are configured to transfer light to and from a surface of a tissue site, such as a skin surface or a surface in a body cavity.

The optical members may transfer emitted light from a light source to the tissue and transfer collected light to a detector. The light source and detector may be any type of light source and detector herein disclosed.

In other examples, the disclosure may not be limited to the conditions which are applied in the previous description. In the following, some other examples are described.

In some examples, it is advantageous to detect light not only from one light source at a time, but also other wavelengths simultaneously. The detector in Figures 1-10 may be replaced by a means to spectrally resolve the light that is emitted back from the fibre, for example by using a spectrometer, or multiple wavelength-selective optical filters and detectors.

In some examples, the aperture or apertures in the mirror is an area on the mirror that is transparent, while the rest of the surface of the mirror is coated with a reflecting material.

In some examples, the aperture or apertures in the mirror is a hole in the mirror, as illustrated in Fig. 9.

In some examples, the aperture or apertures in the mirror is a slit in the mirror, or two or more separate mirrors that form one or multiple slits, as illustrated in Fig. 10.

In the following section basic principles related to the system according to the disclosure will be described wherein the description is based on an exemplary system with three diagnostic light sources and six optical member, such as six modules each comprising three diagnostic light sources coupled to a single optical member, preferably optical fibres.

By a reaction or treatment site we mean in the present context a site where the treatment takes place, such a photothermal therapy, or where a photodynamically active compound may react in a tumour when subject to therapy radiation e.g. conducted by optical members being forwarded through e.g. the lumen of injection needles or a catheter which are placed in the tumour. These optical members may then be fixed in the reaction site. Then the optical members may be moved forward to arrive outside the distal end of the needle or catheter. The same optical member is used continuously during the treatment for integrated diagnostics and dosimetry as well as to avoid that the patient be subjected to multiple pricks.

Preferably the diagnostic light sources are lasers and/or light emitting diodes out of which one is of the same wavelength as the lasers utilised for the laser irradiation for photothermal therapy or photodynamic tumour therapy but could be of lower output power. Suitable filters can be arranged to be inserted into the light path in order to secure that the correct dynamic range is utilised for all measurement tasks and in order to prevent saturation of the radiation detector.

Certain of the diagnostic light sources are utilised in order to study how radiation of the corresponding wavelength is penetrating through the tissue of the tumour at the treatment site.

When radiation from a radiation source is transmitted through the particular optical member via the above described arrangements into the tissue, one of the optical members, functions as a transmitter into the tissue site, such as the tumour, and the other, in this example, five optical fibres in the tissue site, such as the tumour, may act as receivers and collect the diffuse flux of radiation reaching them. The optical members transmit the collected light and emits the light so that it can be detected by at least one detector, as described above, and five different light intensities can be recorded for each wavelength.

As an alternative to a specific wavelength, light from an optically broad light source such, as a white light source, and/or broadband light emitting diodes and/or line light sources may be coupled into the particular active optical member. On passage through the tissue to the optical member in the patient, the well-defined spectral distribution of the optical source may be modified by the tissue absorption. Then, oxygenated blood yields a different signature than non-oxygenated blood, allowing a tomographic determination of the oxygen distribution utilising the thirty different spectral distributions which are read out, five spectra at a time in the six possible different constellations. Such a determination of the oxygenation in the tumour is important, since the PDT process requires access to oxygen in the tissue.

Finally, in the case of one of the light sources being able to induce fluorescence in the tissue, and a sensitizer administered to the tissue displays a characteristic fluorescence distribution shifted towards longer wavelengths. The strength of the corresponding signal allows an approximate quantification of the level of the sensitizer in the tissue. For certain substances, the red light used for the light propagation studies may be used to induce red or near-infrared fluorescence. This fluorescence penetrates through the tissue to the tips of the receiving optical member, and is displayed simultaneously as spectra obtained by one of the detectors. A tomographic calculation of the sensitizer distribution may be performed based on in total thirty measurement values at each measurement occasion.

If the tips of the member in addition are treated with a material, the fluorescence properties of which are temperature dependent, sharp fluorescence lines are obtained upon excitation, and the intensity of these lines and their relative strength depend on the temperature at the tip of the optical member being employed for treatment. Examples of such materials are salts of the transition metals or the rare earth metals. Hence, also the temperature can be measured at the six positions of the six optical members, one at a time or simultaneously. The measured temperatures may be utilised to find out if blood coagulation with an associated light attenuation has occurred at the tip of the optical member and for studies regarding the utilisation of possible synergy effects between PDT and thermal interaction. Since the lines obtained are sharp, they may easily be extracted from the more broad-banded endogenous fluorescence distribution from the tissue.

After diagnostic measurements and calculations have been performed, the optical members coupled to the patient can be utilised for therapy by switching off the diagnostic light sources and switching on the therapeutic light sources, so that therapeutic light sources are coupled to the patient optical members. The therapeutic light sources are preferably laser sources with a wavelength, chosen to match the absorption band of the sensitizer. At the photodynamic tumour treatment, a dye laser or a diode laser is preferably used, with a wavelength which is selected with regard to the sensitizer employed. For e.g. Photofrin^{®} the wavelength is 630 nm, for o-aminolevulinic acid (ALA) it is 635 nm and for phthalocyanines it is around 670 nm, several other sensitizers exist having such characteristic wavelengths. The individual lasers are regulated during the treatment to a desirable individual output power. If desired, they may have built-in or external monitoring detectors.

The therapeutic treatment may be interrupted and new diagnostic data may be processed in an interactive method until an optimal treatment has been reached. This method may include synergy between PDT and hyperthermia, where an increased temperature is reached at increased fluxes of laser radiation. The whole process may be controlled using a control unit, such as a computer, which does not only perform all the calculations but also is utilised for regulation and control of the system. The present invention has been described above with reference to specific examples. However, other examples than the above described are equally possible within the scope of the disclosure. Different method steps than those described above may be provided. The different features of the invention may be combined in other combinations than those described. The scope of the invention is only limited by the appended patent claims.

The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases and disjunctively present in other cases.

## Claims

1. A system for diagnosis of a subject, the system comprising:
at least one diagnostic light source (1) for emission of diagnostic light within a wavelength range of infrared, visible or ultraviolet light, the diagnostic light source emitting at least one light beam;
at least one light detector (3), for detection of light; and
a plurality of optical members (2) adapted to conduct light to and/or from a tissue site of the subject, whereby the distal ends of the plurality of optical members are configured to be positionable at different locations of the tissue site in order to enable an effective diagnosis and/or treatment,
each of the optical members being configured so that at least one of the light beams from the at least one diagnostic light source is coupled into the proximal end of the plurality of optical members by means of at least one focussing optical component (6),
and the diagnostic light transmitted back from the tissue being emitted from the proximal end of the plurality of optical members member having a higher numerical aperture (NA) than the focussed beam coupled into the optical members providing an emission at least partially in a different angular sector than an angular sector of the focussed light beam, so that the diagnostic light scattered back from the tissue is detected by the at least one light detector;
**characterised by** the plurality of optical members are configured to be interstitially arranged in tissue.

2. The system of claim 1, further configured for interactive photodynamic or photothermal therapy, and comprising at least one therapeutic light source for emission of therapeutic light within a wavelength range of infrared, visible or ultraviolet light, said therapeutic light source emitting at least one light beam which is couples into the proximal end of the optical member by means of the focussing optical component.

3. The system of claim 2, wherein the diagnostic light source is the same as the therapeutic light source; or wherein the wavelength range of the diagnostic light source is the same as the wavelength range of the therapeutic light source.

4. The system according to any of claims 1 to 3, wherein a reflective member is used for coupling the light from the proximal end of the optical member to the at least one detector.

5. The system of claim 4, wherein the reflective member has at least one aperture for the diagnostic light to be transmitted through when travelling between the at least one diagnostic light source and the proximal end of the plurality of optical members.

6. The system of claim 5, wherein the aperture is at least one hole or a slit.

7. The system of any of claims 1 to 6, wherein one of the plurality of optical members is a transmission member used for transmitting the diagnostic light to the tissue site and at least two other optical members of the plurality of optical members are receiving members for receiving backscattered light from the tissue site for detection.

8. The system of claim 7, wherein the transmission member is sequentially selected between the plurality of optical members.

9. The system of any of claims 1 to 8, comprising a plurality of modules, wherein each module comprises a light emitting part comprising at least one of the at least one diagnostic light source, a light detecting part comprising at least one of the at least one detector, one of the at least one focussing optical component, and one of the plurality of optical members.

10. The system of any of claims 8 to 9, wherein the transmission member is sequentially selected between the plurality of optical members by sequentially switching on and off the light emitting part of the plurality of modules.

11. The system of any of claims 1 to 10, having an open beam path between the at least one light source and the proximal end of the plurality of optical members, and between the proximal end of the plurality of optical members and the at least one light detector.

12. The system of any of claims 1 to 11, wherein the plurality of optical members configured to be arranged on the tissue site to perform spatially resolved measurements.

13. The system of any of claims 1 to 12, wherein at least one second focusing element is arranged in front of the at least one detector.

## Patentansprüche

1. Ein System zur Diagnose eines Patienten, wobei das System Folgendes umfasst:
mindestens eine Diagnoselichtquelle (1) zur Aussendung von Diagnoselicht in den Wellenlängenbereichen infrarotes, sichtbares oder ultraviolettes Licht, wobei die Diagnoselichtquelle mindestens einen Lichtstrahl aussendet,
mindestens einem Lichtsensor (3) zur Erfassung von Licht und
einer Mehrzahl optischer Elemente (2), die zur Leitung von Licht an und/oder von einem Gewebebereich des Patienten geeignet sind,
wobei die distalen Enden der Mehrzahl optischer Elemente so ausgelegt sind, dass sie an unterschiedlichen Stellen des Gewebebereichs positioniert werden können, um eine wirksame Diagnose und/oder Behandlung zu ermöglichen, jedes dieser optischen Elemente so konfiguriert ist, dass mindestens einer der Lichtstrahlen von mindestens einer Diagnoselichtquelle in ein proximales Ende der Mehrzahl optischer Elemente durch mindestens ein fokussierendes optisches Bauteil (6) eingekoppelt wird und das vom Gewebebereich zurückgeworfene und vom proximalen Ende der Mehrzahl optischer Elemente ausgestrahlte Licht eine höhere numerische Apertur (NA) im Vergleich zum in die optischen Elemente eingekoppelten fokussierten Lichtstrahl besitzt und eine Emission in einem mindestens teilweise unterschiedlichen Winkelbereich als ein Winkelbereich des fokussierten Lichtstrahls bewirkt, so dass das vom Gewebe zurückgeworfene Licht von dem mindestens einen Lichtsensor erfasst wird, **dadurch gekennzeichnet, dass** die Mehrzahl optischer Elemente zur interstitiellen Anordnung im Gewebe ausgelegt ist.

2. Das System gemäß Anspruch 1, das weiterhin für eine fotodynamische oder fotothermale Behandlung ausgelegt ist und mindestens eine Therapielichtquelle zur Emission von Therapielicht in den Wellenlängenbereichen infrarotes, sichtbares oder ultraviolettes Licht, wobei besagte Therapielichtquelle mindestens einen Lichtstrahl aussendet, der in das proximale Ende des optischen Elements mittels des fokussierenden optischen Bauteils eingekoppelt wird.

3. Das System gemäß Anspruch 2, wobei die Diagnoselichtquelle dieselbe wie die Therapielichtquelle ist oder wobei der Wellenlängenbereich der Diagnoselichtquelle derselbe wie der Wellenlängenbereich der Therapielichtquelle ist.

4. Das System gemäß einem der Ansprüche 1 bis 3, wobei ein reflektierendes Element zur Einkopplung des Lichts vom proximalen Ende des optischen Elements in den mindestens einen Sensor verwendet wird.

5. Das System gemäß Anspruch 4, wobei das reflektierende Element mindestens eine Apertur für das zu übermittelnde Diagnoselicht aufweist, wenn dieses zwischen der mindestens einen Diagnoselichtquelle und dem proximalen Ende der Mehrzahl optischer Elemente wandert.

6. Das System gemäß Anspruch 5, wobei die Apertur mindestens einem Loch oder einem Schlitz entspricht.

7. Das System gemäß einem der Ansprüche 1 bis 6, wobei eines aus der Mehrzahl optischer Elemente ein Übertragungsorgan zur Übertragung des Diagnoselichts an den Gewebebereich ist und mindestens zwei andere optische Elemente aus der Mehrzahl optischer Elemente auffangende Organe zum Auffangen des vom Gewebebereich zwecks Erfassung zurückgeworfenen Lichts sind.

8. Das System gemäß Anspruch 7, wobei das Übertragungsorgan sequentiell aus der Mehrzahl optischer Elemente gewählt wird.

9. Das System gemäß einem der Ansprüche 1 bis 8 mit einer Mehrzahl von Modulen, wobei jedes Modul einen lichtemittierenden Teil mit mindestens einer der mindestens einen Diagnoselichtquelle umfasst, einen lichterfassenden Teil mit mindestens einem der mindestens einen Sensoren, einem der mindestens einen fokussierenden optischen Komponente und einem aus der Mehrzahl optischer Elemente.

10. Das System gemäß einem der Ansprüche 8 bis 9, wobei das Übertragungsorgan sequentiell zwischen der Mehrzahl optischer Elemente gewählt wird, indem der lichtemittierende Teil aus der Mehrzahl von Modulen sequentiell ein- und ausgeschaltet wird.

11. Das System gemäß einem der Ansprüche 1 bis 10 mit einem offenen Strahlengang zwischen der mindestens einen Lichtquelle und dem proximalen Ende der Mehrzahl optischer Elemente und zwischen dem proximalen Ende der Mehrzahl optischer Elemente und dem mindestens einen Lichtsensor.

12. Das System gemäß einem der Ansprüche 1 bis 11, wobei die Mehrzahl der optischen Elemente so ausgelegt ist, dass sie im Gewebebereich angeordnet werden kann, um räumlich aufgelöste Messungen vorzunehmen.

13. Das System gemäß einem der Ansprüche 1 bis 12, wobei mindestens ein zweites fokussierendes Element vor dem mindestens einen Sensor angeordnet ist.

## Revendications

1. Système destiné au diagnostic d'un sujet, le système comprenant :
au moins une source de lumière de diagnostic (1) destinée à une émission de lumière de diagnostic dans une plage de longueurs d'onde de lumière infrarouge, visible ou ultraviolette, la source de lumière de diagnostic émettant au moins un faisceau lumineux ;
au moins un détecteur de lumière (3), destiné à une détection de lumière ; et
une pluralité d'éléments optiques (2) conçus pour acheminer de la lumière vers et/ou depuis un site de tissu du sujet, grâce à quoi les extrémités distales de la pluralité d'éléments optiques sont configurées pour pouvoir être positionnées à des positions différentes du site de tissu de manière à permettre un diagnostic et/ou un traitement efficaces,
chacun des éléments optiques étant configuré de sorte qu'au moins un des faisceaux lumineux provenant de la au moins une source de lumière de diagnostic est couplé dans l'extrémité proximale de la pluralité d'éléments optiques au moyen d'au moins un composant optique de focalisation (6),
et la lumière de diagnostic renvoyée par le tissu étant émise depuis l'extrémité proximale de la pluralité d'éléments optiques présentant une ouverture numérique (ON) plus grande que le faisceau focalisé couplé dans les éléments optiques fournissant une émission au moins en partie dans un secteur angulaire différent d'un secteur angulaire du faisceau lumineux focalisé, de sorte que la lumière de diagnostic rétrodiffusée provenant du tissu est détectée par le au moins un détecteur ;
**caractérisé par le fait que** la pluralité d'éléments optiques sont configurés pour être agencés de manière interstitielle dans un tissu.

2. Système selon la revendication 1, en outre configuré pour une thérapie photothermique ou photodynamique, et comprenant au moins une source de lumière thérapeutique destinée à une émission de lumière thérapeutique dans une plage de longueurs d'onde de lumière infrarouge, visible ou ultraviolette, ladite source de lumière thérapeutique émettant au moins un faisceau lumineux lequel est couplé dans l'extrémité proximale de l'élément optique au moyen du composant optique de focalisation.

3. Système selon la revendication 2, dans lequel la source de lumière de diagnostic est la même que la source de lumière thérapeutique ; ou dans lequel la plage de longueurs d'onde de la source de lumière de diagnostic est la même que la plage de longueurs d'onde de la source de lumière thérapeutique.

4. Système selon l'une quelconque des revendications 1 à 3, dans lequel un élément réfléchissant est utilisé pour coupler la lumière de l'extrémité proximale de l'élément optique au au moins un détecteur.

5. Système selon la revendication 4, dans lequel l'élément réfléchissant présente au moins une ouverture par laquelle la lumière de diagnostic passe lors de son parcours entre la au moins une source de lumière de diagnostic et l'extrémité proximale de la pluralité d'éléments optiques.

6. Système selon la revendication 5, dans lequel l'ouverture est au moins un orifice ou une fente.

7. Système selon l'une quelconque des revendications 1 à 6, dans lequel un élément parmi la pluralité d'éléments optiques est un élément de transmission utilisé pour transmettre la lumière de diagnostic au site de tissu et au moins deux autres éléments optiques parmi la pluralité d'éléments optiques sont des éléments de réception destinés à recevoir la lumière rétrodiffusée provenant du site de tissu à des fins de détection.

8. Système selon la revendication 7, dans lequel l'élément de transmission est successivement sélectionné entre la pluralité d'éléments optiques.

9. Système selon l'une quelconque des revendications 1 à 8, comprenant une pluralité de modules, où chaque module comprend une partie d'émission de lumière comprenant au moins l'une de la au moins une source de lumière de diagnostic, une partie de détection de lumière comprenant au moins l'un du au moins un détecteur, l'un du au moins un composant optique de focalisation, et l'un de la pluralité d'éléments optiques.

10. Système selon l'une quelconque des revendications 8 et 9, dans lequel l'élément de transmission est successivement sélectionné entre la pluralité d'éléments optiques en allumant et en éteignant successivement la partie d'émission de lumière de la pluralité de modules.

11. Système selon l'une quelconque des revendications 1 à 10, présentant un trajet de faisceau ouvert entre la au moins une source de lumière et l'extrémité proximale de la pluralité d'éléments optiques, et entre l'extrémité proximale de la pluralité d'éléments optiques et le au moins un détecteur de lumière.

12. Système selon l'une quelconque des revendications 1 à 11, dans lequel la pluralité d'éléments optiques sont configurés pour être agencés sur le site de tissu afin de réaliser des mesures spatialement résolues.

13. Système selon l'une quelconque des revendications 1 à 12, dans lequel au moins un second élément de focalisation est agencé devant le au moins un détecteur.
